(19) Europäisches Patentamt
European Patent Office
Office européen des brevets

(11) **EP 3 240 404 B1**

(12) **EUROPEAN PATENT SPECIFICATION**

(45) Date of publication and mention
of the grant of the patent:
**05.02.2020 Bulletin 2020/06**

(21) Application number: **15817524.0**

(22) Date of filing: **23.12.2015**

(51) Int Cl.:
*A01N 25/08* $^{(2006.01)}$      *A01N 25/10* $^{(2006.01)}$
*A01N 25/12* $^{(2006.01)}$      *A01N 59/00* $^{(2006.01)}$
*B65D 81/28* $^{(2006.01)}$      *C11D 3/50* $^{(2006.01)}$
*D06M 11/42* $^{(2006.01)}$      *D06M 11/79* $^{(2006.01)}$
*A01P 1/00* $^{(2006.01)}$      *A61F 13/84* $^{(2006.01)}$

(86) International application number:
**PCT/GB2015/054143**

(87) International publication number:
**WO 2016/108044 (07.07.2016 Gazette 2016/27)**

(54) **SUBSTRATES HAVING A FUNCTIONAL CAPABILITY**

SUBSTRATE MIT FUNKTIONELLER KAPAZITÄT

SUBSTRATS AYANT UNE CAPACITÉ FONCTIONNELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2014 GB 201423348**

(43) Date of publication of application:
**08.11.2017 Bulletin 2017/45**

(73) Proprietor: **IMERTECH SAS**
**75015 Paris (FR)**

(72) Inventors:
• **CROUCH, Tarquin**
**Southborough**
**Kent TN4 0NL (GB)**

• **BIRD, Michael**
**St Austell**
**Cornwall PL25 5HW (GB)**

(74) Representative: **Haseltine Lake Kempner LLP**
**Redcliff Quay**
**120 Redcliff Street**
**Bristol BS1 6HU (GB)**

(56) References cited:
**WO-A1-00/64259**      **WO-A1-2006/114385**
**WO-A1-2009/058707**      **WO-A1-2012/114130**
**WO-A2-2008/150867**      **KR-A- 20120 108 719**

**Description**

**TECHNICAL FIELD**

The present invention is directed to a surface-mineralized substrate having an enhanced functional capability, for example, enhanced antibacterial activity, to a method of making the surface-mineralized substrate, and to an article of manufacture, for example, packaging for fresh produce, comprising or formed from or of the surface-mineralized substrate.

**BACKGROUND OF THE INVENTION**

Antibacterial materials and products have been widely and rapidly accepted by general consumers. Antibacterial products created by incorporating an antibacterial agent can suppress the growth of bacteria on the surfaces of products when conditions exist where growth can occur.

Antibacterial products have been used in plastics, coating materials, ceramics, natural and artificial leather, stainless steel, rubber, etc.

Incorporating active agents into mineral-based fillers can enhance antimicrobial capabilities of the finished products while additionally fulfilling, and without compromising, other filler functions. For example, US-A-2010/0260866 describes the incorporation of modified mineral-based fillers into substrates such as plastics materials by compounding such that the modified mineral-based filler is dispersed throughout the substrate.

WO 2008/150867 discloses substrates which have been surface-mineralized with inorganic particles such as silver particles.

There is an ongoing need to develop new and useful products having enhanced antibacterial properties and the like, and, moreover, products which are environmentally and economically attractive, e.g., use less active agent to achieve the same or better antibacterial effect. There is also an ongoing need to increase shelf-life in order to reduce food wastage.

**SUMMARY OF THE INVENTION**

According to a first aspect, and in accordance with claim 1, there is provided a surface-mineralized substrate having enhanced antibacterial activity, the substrate comprising an inorganic particulate material embedded in a surface region thereof and/or attached to a surface thereof, wherein the inorganic particulate material has an antibacterial capability, wherein the surface-mineralized substrate has an antibacterial activity enhancement factor (AAEF) of at least 1.5, wherein AAEF = R/W, where R is the antibacterial activity of the surface-mineralized substrate according to ISO 22196:2007 for *Pseudomonas aeruginosa,* and W is the % by weight of inorganic particulate material embedded in the surface region and/or attached to the surface of the surface-mineralized substrate, based on the total weight of the surface mineralized substrate. W is from 0.01 to 0.25.

According to a second aspect, and in accordance with claim 2, there is provided a surface-mineralized substrate having enhanced antibacterial activity, the substrate comprising an inorganic particulate material embedded in a surface region thereof and/or attached to a surface thereof, wherein the inorganic particulate material has an antibacterial capability, and wherein the surface-mineralized substrate has an antibacterial activity, R, of at least about 3.0 according to ISO 22196:2007 for *Pseudomonas aeruginosa* when the substrate comprises from 0.02 to 0.10 % by weight of inorganic particulate material in the surface region thereof and/or attached to the surface thereof, based on the total weight of the surface-mineralized substrate.

According to a third aspect, and in accordance with claim 3, there is provided a surface-mineralized substrate having an inorganic particulate material embedded in a surface region thereof and/or attached to a surface thereof, wherein the surface-mineralized substrate has at least one functional capability, wherein the substrate comprises from 0.01 to 0.25 % by weight of the inorganic particulate material in the surface region thereof and/or attached to the surface thereof, wherein the at least one functional capability is selected from:

biocidal (including herbicidal, fungicidal and/or pesticidal) activity; and/or
biocide (including herbicide, fungicide and pesticide) release,

wherein the at least one functional capability is a property of the inorganic particulate and/or is a property of an active agent associated with the inorganic particulate material in the surface region thereof and/or attached to the surface thereof, with the proviso that any release and absorbance capability are mutually exclusive.

According to a fourth aspect, there is provided an article of manufacture, for example, packaging for fresh produce, comprising or formed from or of a surface-mineralized substrate according to the first, second or third aspects.

According to a fifth aspect, there is provided method suitable for making a surface-mineralized substrate according to the first, second or third aspects, the method comprising applying an inorganic particulate material having an antibacterial capability to the surface of a substrate, wherein the substrate has a surface region which is embeddable with the inorganic particulate material and/or a surface to which the inorganic particulate material is attachable, and wherein the inorganic particulate becomes embedded in the surface region of the substrate and/or attached to the surface of the substrate upon further processing.

According to sixth aspect, there is provided a method for improving the antibacterial activity of a substrate suitable for use in or as packaging for fresh produce, said method comprising surface-mineralizing the substrate in accordance with the method according to the fifth aspect.

According to a seventh aspect, there is provided a method for improving the shelf-life of fresh poultry meat, said method comprising packaging fresh poultry meat in packaging comprising or formed from or of a surface-mineralized substrate according to the first, second or third aspects.

In the above aspects, the inorganic particulate material is associated with an antibacterial agent, the antibacterial agent being silver.

## BRIEF DESCRIPTION OF THE DRAWINGS

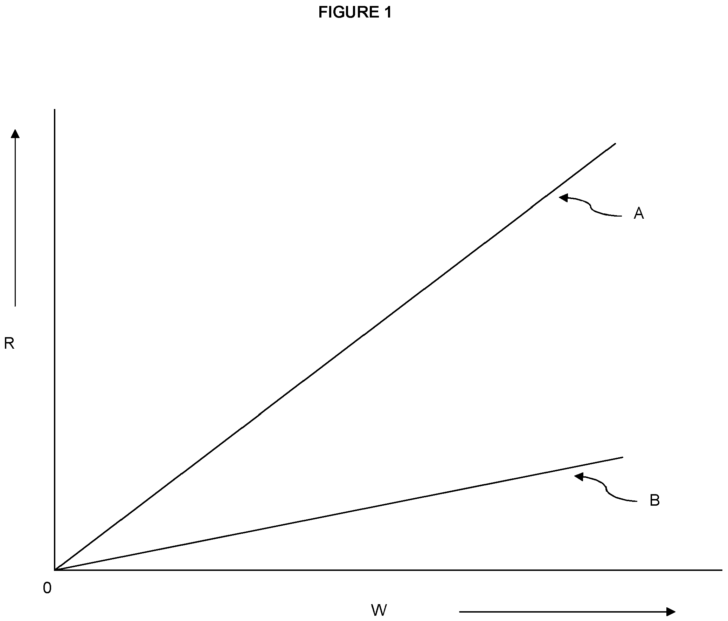

FIG. 1. is a graphical representation of the relationship R/W, wherein R is antibacterial activity, and W is the % by weight of inorganic particulate material having an antibacterial capability.

## DETAILED DESCRIPTION OF THE INVENTION

It has surprisingly been found that the antibacterial activity of a substrate may be enhanced by surface mineralizing the substrate, i.e., by having an inorganic particulate material having an antibacterial capability embedded in a surface region of the substrate and/or attached to a surface of the substrate. By "enhanced" is meant that antibacterial activity of the substrate is either improved compared to a substrate made by compounding the active agent into the substrate, or that a comparable level of activity is obtainable using less active agent, or even an enhanced level of activity is obtainable using less active agent, compared to a substrate made by compounding the active agent into the substrate. This is of significant commercial benefit because the surface-mineralized substrate is both environmentally and economically attractive because less active agent is required to obtain a desired level of antibacterial protection. Also, recognising the advantages in antibacterial activity obtainable by having the substrate surface-mineralized, this finding may be extended to other functional capabilities that may benefit from having the active agent at the surface such as, for example, more broadly to biocidal activity (including herbicidal, fungicidal and/or pesticdial activity); and/or biocide release (including herbicide, fungicide, and pesticide release); and/or fertilizer release; and/or gas release other than odour release; and/or $CO_2$ generation; and/or odour release; and/or liquid or gas absorbance other than odour absorbance; and/or odour absorbance; and/or desiccation.

As used herein, the term "antibacterial" describes a state where growth of bacteria on the surfaces of products is suppressed or describes the effect of an agent which suppresses the growth on the surfaces of products.

As used herein, the terms "antibacterial capability and "antibacterial agent" mean a species which inhibits the growth of bacteria on the surfaces of products.

As used herein, the term "antibacterial activity" means the difference in the logarithm of the viable cell counts found on an antibacterial treated product and an untreated product after inoculation and incubation of bacteria. In accordance with ISO 22196:2007, the antibacterial activity, R, (for *Pseudomonas aeruginosa* (ATCC® 15442™; strain designation PRD-10 [CIP 103467, NCIB 10421, PCI 812])) is calculated as follows:

$$R = (U_t - U_0) - (A_t - U_0) = U_t - A_t \qquad (1)$$

where:

$U_0$ = average of the common logarithm of the number of viable bacteria, in cells/cm$^2$, recovered from the untreated test specimens immediately after inoculation;
$U_t$ = average of the common logarithm of the number of viable bacteria, in cells/cm$^2$, recovered from the untreated test specimens after 24 h; and
$A_t$ = average of the common logarithm of the number of viable bacteria, in cells/cm$^2$, recovered from the treated test specimens after 24 h.

The number of viable bacteria is calculated as follows:

$$N = (100 \times C \times D \times V)/A \qquad\qquad (2)$$

where:

N = number of viable bacteria recovered per $cm^2$ per test specimen;
C = average plate count for the duplicate plates;
D = dilution factor for the plates counted;
V = volume, in ml, of Neutralizing solution added to the specimen; and
A = surface area, in $mm^2$, of the cover film.

By "surface-mineralized" is meant that the substrate is capable of having an inorganic particulate material (i.e., mineral) having an antibacterial capability embedded in a surface region of the substrate or attached to a surface of the substrate by applying the inorganic particulate material having an antibacterial capability to the surface of the substrate under suitable conditions.

As used herein, the term "surface region" means a region extending from the surface of the substrate into the body of the substrate. The inorganic particulate material having an antibacterial capability may be partially or wholly embedded in the surface region. By "partially" is meant that a portion of the inorganic particulate and/or individual particles thereof are not wholly covered in the matrix material, e.g., polymer, of the substrate. In certain embodiments, at least a portion of the inorganic particulate material and/or individual particles thereof are partially embedded in the surface region such that they are exposed to the external environment. In certain embodiments, at least a portion of the inorganic particulate and/or individual particles are sized such that they are at least partially exposed to the external environment following application to the surface of the substrate. In embodiments in which the inorganic particulate material having an antibacterial capability is partially embedded and/or attached to the surface of the substrate, a major proportion (i.e., at least 50 %) of the total surface area of the inorganic particulate material may be exposed to the external environment following application to the surface of the substrate.

As used herein, the term "enhanced antibacterial activity" means that the antibacterial activity of the substrate is either improved compared to a substrate made by compounding the active agent (e.g., an inorganic particulate material having an antibacterial capability), or that a comparable level of activity is obtainable using less active agent.

For the avoidance of doubt, the inorganic particulate material having an antibacterial capability which is embedded in a surface region of the substrate or is attached to a surface of the substrate is not comprised within a coating or coating layer or film layer applied to a position on a surface of the substrate.

Further, the inorganic particulate material having an antibacterial capability is not prepared as a coating by making a dispersion (e.g., a liquid or fluid dispersion) of the inorganic particulate material in a curable or other type of coating which is then applied onto the surface of a substrate.

In certain embodiments, the inorganic particulate material, having an antibacterial capability or other functional capability, is not prepared as a fluid which is then applied to the substrate.

Advantageously, in certain embodiments, the substrate is surface-mineralized during manufacture of the substrate, e.g., following extrusion of a polymeric substrate. Such embodiments are described in greater detail below.

In certain embodiments, for example, embodiments in which the substrate is a cellulose-derived substrate, or a textile, including nonwovens, the antibacterial activity of the surface mineralized substrate may be determined in accordance with ISO 20743:2007. A method suitable for determining fungicidal resistance of paper products is ASTM D2574.

In certain embodiments, the surface-mineralized substrate has an antibacterial activity enhancement factor, AAEF, of at least about 1.5, wherein:

$$AAEF = R/W \qquad\qquad (4)$$

where R is the antibacterial activity of the surface-mineralized substrate according to ISO 22196:2007 for *Pseudomonas aeruginosa* (ATCC® 15442™ (strain designation PRD-10 [CIP 103467, NCIB 10421, PCI 812])), and W is the % by weight of inorganic particulate material having an antibacterial capability embedded in the surface region and/or attached to the surface of the surface-mineralized substrate, based on the total weight of the surface-mineralized substrate.

Conceptually, the AAEF reflects the relationship between the antibacterial activity of the surface-mineralized substrate and the % by weight of inorganic particulate material having an antibacterial capability. This may be visualised graphically, as show in Fig. 1. In the graph depicted in Fig. 1., the y-axis is antibacterial activity, R, and the x-axis is the % by weight, W, of inorganic particulate material having an antibacterial capability (and inclusive of any antibacterial agent present). The upper line, A, representative of a surface-mineralized substrate, has a steeper gradient than lower line, B, representative of a compounded substrate. Visually, it can be seen that the surface-mineralized substrate has an enhanced antibacterial activity (i.e., steeper gradient of the line of R/W) compared to a mineral compounded into a substrate). In certain embodiments, the surface-mineralized substrate has an AAEF of at least about 2, or at least about 5, or at least about 10, or at least about 20, or at least about 30, or at least about 40, or at least about 50, or at least about 60, or at least about 70, or at least about 80, or at least about 90, or at least about 100, or at least about 110, or at least about 120. In such embodiments, W is from about 0.01 to about 0.25 % by weight, for example, from about 0.02 to about 0.25 % by weight, or from about 0.02 to about 0.10 % by weight, or from about 0.02 to about 0.09 % by weight, or from about 0.02 to about 0.08 % by weight, or from about 0.02 to about 0.07 % by weight, or from about 0.02 to about 0.06 % by weight, or from about 0.03 to about 0.05 % by weight. In such embodiments, R is at least about 1.5, or at least about 2.0, or at least about 2.5, or at least about 3.0, or at least about 3.5, or at least about 4.0, or at least about 4.5, or at least about 5.0, or at least about 5.5. In certain embodiments, R is at least about 3.0, or at least about 3.5 and W is from 0.02 to about 0.10 % by weight, or from about 0.02 to about 0.08 % by weight, or from about 0.03 to about 0.06 % by weight.

Also described is a surface-mineralized substrate having an AAEF of at least about 2.5, where R is the antibacterial activity of the surface-mineralized substrate according to ISO 22196:2007 for *Staphylococcus aureus* (ATCC® 6538™ (strain designation: FDA 209)), and W is the % by weight of inorganic particulate material having an antibacterial capability embedded in the surface region and/or attached to the surface of the surface-mineralized substrate, based on the total weight of the surface-mineralized substrate. The surface-mineralized substrate may have an AAEF of at least about 2, or at least about 5, or at least about 10, or at least about 20, or at least about 30, or at least about 40, or at least about 50, or at least about 60, or at least about 70, or at least about 80, or at least about 90, or at least about 100, or at least about 110, or at least about 120. In such embodiments, W may be from about 0.01 to about 3 % by weight, for example, from about 0.01 to about 2 % by weight, or from about 0.02 to about 1.5 % by weight, or from about 0.02 to about 1.0 % by weight, or from about 0.02 to about 0.50 % by weight, or from about 0.02 to about 0.25 % by weight, or from about 0.02 to about 0.10 % by weight, or from about 0.02 to about 0.09 % by weight, or from about 0.02 to about 0.08 % by weight, or from about 0.02 to about 0.07 % by weight, or from about 0.02 to about 0.06 % by weight, or from about 0.03 to about 0.05 % by weight. R may be at least about 1.0, or at least about 1.5, or at least about 2.0, or at least about 2.5, or at least about 3.0, or at least about 3.5, or at least about 4.0, or at least about 4.5, or at least about 5.0, or at least about 5.5. R may be at least about 4.5, or at least about 5.0, or at least about 5.5, and W is from about 0.02 to about 1.0 % by weight, or from about 0.02 to about 0.5 % by weight, or from about 0.02 to about 0.10 % by weight, or from about 0.02 to about 0.08 % by weight, or from about 0.03 to about 0.06 % by weight.

In certain embodiments, R is no greater than about 20.0, for example, no greater than about 15.0, for example, no greater than about 10.0, for example, no greater than about 9.0, or no greater than about 8.0, or no greater than about 7.0.

Also described is a surface-mineralized substrate having a campylobacter reduction factor, CRF, wherein:

$$CRF = W \times (cfu/g) \qquad\qquad (5)$$

and wherein CRF is no greater than about 5000 when cfu/g is about 1000 or no greater than about 1000, where W is the % by weight of inorganic particulate material having an antibacterial capability embedded in the surface region and/or attached to the surface of the surface-mineralized substrate, based on the total weight of the surface-mineralized substrate, cfu/g is colony formation units per gram (i.e., the amount of campylobacter present on poultry measured in terms of the number of bacteria on a given weight of skin). In this respect, the UK Food Standards Agency has established a Joint Working Group (JWG) to tackle campylobacter in poultry. The JWG has agreed a target to reduce the percentage of chickens with more than 1000 cfu/g at the end of the slaughter process to 10 % by 2014. See: http://www.food.gov.uk/sites/default/files/multimedia/pdfs/publication/campylobacter-factsheet-the-science.pdf

The CRF may be no greater than about 4000 when cfu/g is about 1000, for example, no greater than about 3000, or no greater than about 2000, or no greater than about 1000, or no greater than about 750, or no greater than about 500, or no greater than about 400, or no greater than about 300, or no greater than about 200, or no greater than about 100, or no greater than about 50. Thus, the CRF reflects that a relatively low amount of inorganic particulate material having antibacterial capability and, when present, antibacterial agent, is required to control campylobacter levels in accordance with 2015 targets, when the inorganic particulate material having antibacterial capability and, when present, antibacterial agent, reside in the surface region of the substrate, i.e., are embedded in or attached to a surface of the substrate.

Also described is a surface-mineralized substrate comprising an inorganic particulate material having an antibacterial capability embedded in a surface region thereof and/or attached to a surface thereof and an antibacterial activity, R, of at least about 2.0 according to ISO 22196:2007 for *Pseudomonas aeruginosa* (ATCC® 15442™ (strain designation PRD-10 [CIP 103467, NCIB 10421, PCI 812])) when the substrate comprises no more than about 3.0 % by weight of inorganic particulate material having an antibacterial capability (including any antibacterial agent when present) in the surface region thereof and/or attached to the surface thereof, based on the total weight of the surface-mineralized substrate.

In certain embodiments, the surface-mineralized substrate has an antibacterial activity, R, of at least 3.0 according to ISO 22196:2007 for *Pseudomonas aeruginosa* (ATCC® 15442™ (strain designation PRD-10 [CIP 103467, NCIB 10421, PCI 812])) when the substrate comprises from 0.02 to 0.10 % by weight of inorganic particulate material having an antibacterial capability, for example, from about 0.02 to about 0.09 % by weight, of from about 0.02 to about 0.08 % by weight, or from about 0.02 to about 0.07 % by weight, or from about 0.03 % to about 0.06 % by weight, or from about 0.03 to about 0.05 % by weight. In such embodiments, R may be at least about 3.5, or at least about 4.0. In certain embodiments, R is at least 3.0 when the substrate comprises no more than about 0.10 % by weight particulate material having an antibacterial capability in the in the surface region thereof and/or attached to a surface thereof.

Also described is a surface-mineralized substrate comprising an inorganic particulate material having an antibacterial capability embedded in a surface region thereof and/or attached to a surface thereof and an antibacterial activity, R, of at least about 3.0 according to ISO 22196:2007 for *Staphylococcus aureus* (ATCC® 6538™ (strain designation: FDA 209)) when the substrate comprises no more than about 3.0 % by weight of inorganic particulate material having an antibacterial capability (including any antibacterial agent when present) in the surface region thereof and/or attached to the surface thereof, based on the total weight of the surface-mineralized substrate.

The surface-mineralized substrate may have an antibacterial activity, R, of at least about 4.0 according to ISO 22196:2007 for *Staphylococcus aureus* (ATCC® 6538™ (strain designation: FDA 209)) when the substrate comprises no more than about 2.0 % by weight of inorganic particulate material having an antibacterial capability, for example, no more than about 1.5 % by weight, or no more than about 1.0 % weight, or no more than about 0.5 % by weight, or no more than about 0.25 % by weight, or no more than about 0.1 % by weight, or from about 0.01 to about 0.09 % by weight, of from about 0.01 to about 0.08 % by weight, or from about 0.02 to about 0.07 % by weight, or from about 0.03 % to about 0.06 % by weight, or from about 0.03 to about 0.05 % by weight. R may be at least about 4.5, or at least about 5.0, or at least about 5.5. R may be at least 4.5 when the substrate comprises no more than about 0.10 % by weight particulate material having an antibacterial capability in the in the surface region thereof and/or attached to a surface thereof.

The amount of inorganic particulate material in the surface region of the surface-mineralized substrate and/or attached to a surface of the surface-mineralized substrate may be determined by ashing the surface-mineralized substrate.

In certain embodiments of the third aspect, the surface-mineralized substrate having an inorganic particulate material embedded in a surface region thereof and/or attached to a surface thereof has at least one functional capability, wherein the substrate comprises from 0.01 to 0.25 % by weight of the inorganic particulate material in the surface region thereof and/or attached to the surface thereof, wherein the at least one functional capability is selected from:

biocidal activity (including herbicidal, fungicidal and/or pesticdial activity); and/or
biocide release (including herbicide, fungicide and pesticide release,

wherein the at least one functional capability is a property of the inorganic particulate material and/or is a property of an active agent associated with the inorganic particulate material in the surface region thereof and/or attached to the surface thereof, with the proviso that any release and absorbance capability are mutually exclusive.

*Inorganic particulate material*

The inorganic particulate material (i.e., mineral-based material) may, for example, be an alkaline earth metal carbonate or sulphate, such as calcium carbonate, magnesium carbonate, dolomite, gypsum, clay, for example, a hydrous kandite clay such as kaolin, halloysite or ball clay, an anhydrous (calcined) kandite clay such as metakaolin or fully calcined kaolin, talc, mica, perlite, diatomaceous earth, vermiculate, magnesium hydroxide, aluminium trihydrate, a metallic oxide, natural or synthetic zeolite, sodium nitrite or nitrate, or combinations thereof.

In certain embodiments, the inorganic particulate material is a mineral-based filler (e.g., a silver doped diatomaceous earth) as described in any of one paragraphs [0016]-[0027] of US-A-2010/0260866.

In certain embodiments, inorganic particulate material having an antibacterial capability is selected from the group consisting of diatomaceous earth, kaolin, mica, vermiculite, perlite, talc, zeolite, and aluminosilicates.

In certain embodiments, the inorganic particulate material is diatomaceous earth. Diatomaceous earth is, in general, a sedimentary biogenic silica deposit comprising the fossilized skeletons of diatoms, one-celled algae-like plants that accumulate in marine or fresh water environments. Honeycomb silica structures generally give diatomaceous earth useful characteristics such as absorptive capacity and surface area, chemical stability, and low bulk density. In one embodiment, diatomaceous earth comprises about 90% $SiO_2$ mixed with other substances. In another embodiment, diatomaceous earth comprises about 90% $SiO_2$, plus various metal oxides, such as but not limited to Al, Fe, Ca, and Mg oxides. In one embodiment, the diatomaceous earth is natural, e.g., unprocessed. The impurities in natural diatomaceous earth, such as clays and organic matters, may provide higher cation exchange capacity. In another embodiment, the diatomaceous earth is calcined. In a further embodiment, the diatomaceous earth is flux calcined. In yet another embodiment, diatomaceous earth is a commercially available super-fine diatomaceous earth product, such as but not limited to Superfloss® available from Imerys. In yet a further embodiment, the diatomaceous earth is CelTiX™, available from Imerys.

In certain embodiments, the inorganic particulate material is perlite. Perlite, in general, identifies any naturally occurring siliceous volcanic rock that can be expanded with heat treatment. In one embodiment, perlite comprises between about 70% and about 74% silica, about 14% alumina, between about 2% and 6% water, and trace impurities. In one embodiment, the perlite is ore. In another embodiment, the perlite is expanded. In a further embodiment, the perlite is a fine perlite. In yet another embodiment, the perlite is Harborlite® 635, a very fine grade of perlite available from Imerys.

In a further embodiment, the inorganic particulate material is at least one clay. In one embodiment, the at least one clay is smectite, which may also be referred to as bentonite clay. Bentonite clay generally comprises absorbent aluminum phyllosilicates, impure clays, and may consist mostly of montmorillonite, $(Na,Ca)_{0.33}(Al,Mg)_2Si_4O_{10}(OH)_2.(H_2O)_n$, which may also be referred to as Fuller's earth. In certain embodiments, the at least one clay is illite clay. Exemplary illite clays include, but are not limited to, gumbelite, hydromica, hydromuscovite, muscovite, and sericite.

In certain embodiments, the inorganic particulate material is kaolin clay, which may also be referred to as china clay or hydrous kaolin. In one embodiment, kaolin clay comprises predominantly mineral kaolinite ($Al_2Si_2O_5(OH)_4$), anhydrous aluminum silicate, and amounts of various impurities. The kaolin clay may be used in any one of various common forms. Exemplary forms of kaolin clays include, but are not limited to, airfloat kaolin clay, water-washed kaolin clay, delaminated kaolin clay, and calcined kaolin clay.

In certain embodiments, the inorganic particulate material is a metallic oxide, for example, a synthetic calcium silicate hydrate ($CaSiO_3$). An exemplary synthetic $CaSiO_3$ is Micro-Cel® E, available from Imerys. In certain embodiments, the inorganic particulate material is vermiculite.

In certain embodiments, the inorganic particulate material is talc. Talc may be used in any of various common forms. In one embodiment, the talc comprises greater than about 90% $Mg_3Si_4O_{10}(OH)_2$ (magnesium silicate hydroxide) and accessory minerals in varying amounts, including, but not limited to, chlorite, serpentine, quartz, tremolite, anthophyllite, and carbonates such as magnesite, dolomite, and calcite. In another embodiment, the talc is platy talc. In a further embodiment, the talc is industrial talc. In yet another embodiment, the talc is tremolitic talc. In still a further embodiment, the inorganic particulate material is a synthetic magnesium silicate hydrate ($MgSiO_3$). An exemplary synthetic $MgSiO_3$ is Celkate® T21, available from Imerys.

In certain embodiments, the at least one mineral-based filler is mica. Mica may be used in any of various common forms. In one embodiment, the mica of the general Formula (I):

$$X_2Y_{4-6}Z_8O_{20}(OH,F)_4 \qquad (6)$$

wherein X may be, but is not limited to, K, Na, Ca, Ba, Rb, or Cs; Y may be, but is not limited to, Al, Mg, Fe, Mn, Cr, Ti, and Li; and, Z may be, but is not limited to, Si, Al, Fe, and Ti.

In certain embodiments, the inorganic particulate material is selected from the group consisting of activated carbon, high aspect ratio Wollastonite, low aspect ratio Wollastonite, amorphous silicas, amorphous aluminas, alumina trihydrate, barite (barium sulfate), bentonite, ground calcium carbonate, precipitated calcium carbonate, calcium sulfate, gypsum, carbon black, clay, chlorite, dolomite, feldspar, graphite, huntite, hydromagnesite, hydrotacite, magnesia, magnesite (magnesium carbonate), magnesium hydroxide, magnetite ($Fe_3O_4$), nepheline syenite, olivine, pseudoboehmites (forms of microcrystalline aluminum hydroxide), pyrophyllite, titania, titanium dioxide (e.g., rutile), Turkish powder, stone powder, pumice, coral sand, natural and synthetic zeolites, and zinc oxide, all of which may be used in any of various common forms.

In certain embodiments, the inorganic particulate material is silica. Silica may be used in any of various common forms. Examples of silica forms include, but are not limited to, ground silica, novoculite silica, precipitated silica, fumed silica, and fumed amorphous silica. In one embodiment, the silica is synthetic silica. Examples of synthetic silicas include, but are not limited to, silica gels, silica colloids, synthetic fused silica, and doped synthetic fused silica. In another embodiment, the silica is an aluminosilicate with the basic structural composition $AlSiO_4$. Exemplary aluminosilicates include, but are not limited to, calcium aluminosilicate, sodium aluminosilicate, potassium aluminosilicate, zeolite, and kyanite.

In certain embodiments, the inorganic particulate material is a mineral powder. Exemplary mineral powders include, but are not limited to, fly ash, low calcium fly ash, Class F fly ash, Class C fly ash, bark ash, bottom ash, pet coke ash, silica fume, condensed silica fume, rice hull ash, slag, air-cooled slag, normal weight slag, lightweight slag, expanded slag, pelletized slag, ground granulated blast furnace slag, sandy volcanic ash, volcanic tuffs, and natural pozzolans.

In certain embodiments, the inorganic particulate is sodium nitrite or sodium nitrate, or any other inorganic particulate which has the capability to donate or generate nitric oxide in the presence of an active agent such as a reducing agent. The reducing agent may be, for example, an enzyme, such as enzymes associated with fresh meat, e.g., fresh red meats, containing myoglobin capable of association with nitric oxide to produce nitroxymyoglobin.

Unless otherwise stated, particle size properties referred to herein for the inorganic particulate materials are as measured by the well known conventional method employed in the art of laser light scattering, using a CILAS 1064 instrument (or by other methods which give essentially the same result). In the laser light scattering technique, the size of particles in powders, suspensions and emulsions may be measured using the diffraction of a laser beam, based on an application of Mie theory. Such a machine provides measurements and a plot of the cumulative percentage by volume of particles having a size, referred to in the art as the 'equivalent spherical diameter' (e.s.d), less than given e.s.d values. The mean particle size $d_{50}$ is the value determined in this way of the particle e.s.d at which there are 50% by volume of the particles which have an equivalent spherical diameter less than that $d_{50}$ value. The term $d_{90}$ is the particle size value less than which there are 90% by volume of the particles.

In certain embodiments, the inorganic particulate has a $d_{50}$ of equal to or less than about 50 $\mu$m, for example, equal to or less than about 35 $\mu$m, or equal to or less than about 20 $\mu$m, or equal to or less than about 15 $\mu$m, or equal to or less than about 10 $\mu$m, or equal to or less than about 5 $\mu$m. In certain embodiments, the inorganic particulate material has a $d_{50}$ of from about 1 and about 20 $\mu$m, for example, from about 1.0 to about 15 $\mu$m, or from about 1.0 to about 10 $\mu$m, or from about 1.0 to about 5 $\mu$m.

As described herein, the amount of inorganic particulate material (including any associated active agent) embedded in a surface region and/or attached to a surface of the substrate is generally no more than about 5.0% by weight, based on the total weight of the surface-mineralized substrate. In certain embodiments, the amount of inorganic particulate material (including any associated active agent) embedded in a surface region and/or attached to a surface of the substrate is no more than about 4.0 % by weight, for example, no more than about 3.0 % by weight, or no more than about 2.0 % by weight, or no more than about 1.5 % by weight, or no more than about 1.0 % weight, or no more than about 0.5 % by weight, or no more than about 0.25 % by weight, or no more than about 0.1 % by weight, or from about 0.01 to about 0.09 % by weight, of from about 0.01 to about 0.08 % by weight, or from about 0.02 to about 0.07 % by weight, or from about 0.03 % to about 0.06 % by weight, or from about 0.03 to about 0.05 % by weight.

In certain embodiments, the surface-mineralized substrate comprises an amount of inorganic particulate material other than in the surface region and/or attached to a surface thereof, for example, an amount of inorganic particulate that is incorporated during compounding of the substrate. In certain embodiments, the surface-mineralized substrate does not contain any inorganic particulate material other than in the surface region and/or attached to a surface thereof.

*Active agents including antibacterial agents*

The inorganic particulate material may have an inherent functional capability, for example, an inherent biocidal activity such as, for example, an inherent antibacterial capability. For example, diatomaceous earth is known to have an inherent antibacterial capability.

In accordance with the first, second and third aspects, the inorganic particulate material is associated with an active agent which provides or enhances at least one functional capability of the surface mineralized substrate. The active agent is an antibacterial agent. In such embodiments, the inorganic particulate material may or may not have an inherent functional capability. In certain embodiments, "associated with" means that the active agent is bound to, or adsorbed by, or retained by, or otherwise interacts with the inorganic particulate material such it is remains part of the surface-mineralized substrate following application of the inorganic particulate material to the surface of the substrate.

Examples of active agent include silver oxides, silver silicates (e.g., silver metasiliate ($Ag_2SiO_3$) and silver orthosilicate ($Ag_4SiO_4$)), silver salts (e.g., silver halogenide, silver nitrate, silver sulfate, silver carboxylates (e.g., silver acetate, silver benzoate, silver carbonate, silver citrate, silver lactate, and silver salicylate)), Hydrogen Peroxide/Silver (such as Accepta 8102 available from Accepta™ Advanced Chemical Technologies), copper oxides, copper salts (e.g., copper sulfide, copper nitrate, copper carbonate, copper sulfate, copper halogenides, and copper carboxylates), zinc oxides, and zinc salts (e.g., zinc sulfide, zinc silicate, zinc acetate, zinc chloride, zinc nitrate, zinc sulfate, zinc gulconate, zinc lactate, zinc oxalate, zinc iodate, and zinc iodide).

The active agent is silver. Other active agents include copper, magnesium, aluminum, niobium, silicon, tantalum, zirconium, cobalt, hafnium, lanthanum, tungsten, calcium, titanium, vanadium, cerium, strontium, tin, and zinc ions.

In certain embodiments, the active agent is a silver ion. In certain e-embodiments, the inorganic particulate material is doped with one or more of the preceding metal ions. In certain embodiments, the inorganic particulate material is diatomaceous earth. In certain embodiments, the inorganic particulate material having an antibacterial capability is silver-doped diatomaceous earth, for example, a silver-doped diatomaceous earth as described in US-A-2010/0260866. In certain embodiments, the silver-doped diatomaceous earth comprises, by weight, from about 25 ppm to about 500 ppm silver, for example, from about 50 ppm to about 300 ppm silver.

Exemplary classes of biocides include, but are not limited to, germicides, bactericides, fungicides, algaeicides, pesticides, rodenticides, avicides, molluscicides, piscicides, insecticides, acaricides, anilides (e.g., tricolcarban), biguanides (e.g., chlorhexidine, alexidine, polymeric biguanides), bishphenols (e.g., triclosan, hexachloophene), halophenpols (PCMX (p-chloro-m-xylenol), heavy metals, phenols and cresols, quarternary ammonium compounds (e.g., cetrimide, benzalkonium chloride, cetylpyridinium chloride) and products to control other arthropods, disinfectants, human hygiene biocidal products, private area and public health disinfectants, veterinary hygiene biocidal products, food and feed area disinfectants, drinking water disinfectants, pest repellants, pest attractants, antifouling products, embalming fluids, taxidermist fluids, and vertebrate control biocides. Exemplary biocides include, but are not limited to, silver acetate, silver carbonate, silver chloride, silver copper zeolite, silver fluoride, silver iodide, colloidal silver nitrate, silver orthophosphate ($Ag_3PO_4$), silver oxide ($Ag_4O_4$), silver salt of partially polymerized mannuronic acid, silver sodium hydrogen zirconium phosphate ($Ag_{0.18}Na_{0.57}H_{0.25}Zr_2(PO_4)_3$), silver thiocyanate, silver thiuronium acrylate co-polymer, silver zeolite, silver zinc zeolite, silver, silver borosilicate, silver magnesium aluminium phosphate zinc 8-quinolinolate, zinc bacitracin, zinc chloride, zinc dehydroabietylammonium 2-ethylhexanoate, zinc dodecyl benzene sulphonate, zinc silicate, zinc sulfate heptahydrate, zinc sulfate, zinc nitrate and anhydrous zinc trichlorophenate ziram.

Also described are active agents which are reducing agents. The reducing agent may be, for example, an enzyme (natural and/or synthetic), such as enzymes associated with fresh meat, e.g., fresh red meats, containing myoglobin capable of association with nitric oxide to produce nitroxymyoglobin. The reducing agent, for example, enzyme, may be present in meat juices associated with the fresh meat.

The active agent, is present in an amount of which will vary according to the type of active agent, its intended function, the type of inorganic particulate material and the substrate which is to be surface-mineralized. In certain embodiments, the amount of active agent is no more than about 10 % by weight, based on the weight of the inorganic particulate material embedded within the surface region of the substrate and/or attached to a surface of the substrate, for example, from about 0.001 % by weight to about 10 % by weight, of from about 0.001 to about 5 % by weight, or from about 0.002 to about 5 % by weight, or from about 0.005 to about 4 % by weight, or from about 0.005 to about 3 % by weight, or from about 0.005 to about 2 % by weight, or from about 0.005 to about 1 % by weight, or from about 0.01 % to about 1 % by weight, or from about 0.05 to about 1 % by weight, or from about 0.1 to about 1 % by weight.

The inorganic particulate material with associated active agent (which is an antibacterial agent) may be prepared by any suitable process whereby the active agent binds to, is adsorbed by, or is retained by, or otherwise interacts with, the inorganic particulate material. Suitable process are described, for example, at paragraphs [0034] to [0047] of US-A-2010/0260866. For example, an exemplary process is one which exchanges ions between a biocidal metal ion (e.g., silver) and the inorganic particulate material (e.g., diatomaceous earth), whereby an aqueous solution of water soluble metal salt (such as silver nitrate) is used for surface adsorption and/or retention, or to cationically modify the inorganic particulate material (e.g., diatomaceous earth) by ionexchange with a biocidal metal ion (e.g., silver ions).

Also described is a surface-mineralized substrate which comprises, in addition to or instead of an inorganic particulate material, a surface-mineralized particulate material or active agent having the functional capability of $CO_2$ generation or emission. The species having such a functional capability when applied to the substrate by the surface-mineralization process described herein, or otherwise included in packaging comprising of formed from or of the surface-mineralized substrate, may be a $CO_2$-producing component. For example, the $CO_2$-producting component may be a carbonate containing species, such as calcium carbonate or sodium bicarbonate which upon dissolution, generates $CO_2$. The dissolution activator may be liquor from the product packaged in the packaging, e.g., meat juices such as chicken liquor, or a food grade or approved liquid activator such as, for example, an acid, e.g., monocalcium phosphate and/or disodium pyrophosphate and the like.

The substrate may be of any suitable form such that it may be used in any application known to the skilled person in which enhanced performance of the application is desired through an increased level of functional capability, for example, an enhanced antibacterial activity. Exemplary applications include food processing and packaging, particularly foodstuffs, more particularly packaging of fresh produce such as uncooked meats, for example, poultry meat, wherein the fresh produce is susceptible to degradation by bacteria and the concomitant problem of extending shelf-life of such produce.

In certain embodiments, the substrate is, or may be processed into, food and beverage containers, storage and bags, food handling equipment, food packaging, food and meat crates, food trays, containers and covers, sealing films therefor, and food wrap.

In certain embodiments, the substrate is a polymeric substrate, for example, a plastic, which is, or may be processed into, and article selected from articles for building and construction, household items and furnishings, electrical and electronic parts, coatings and laminates, transportation and recreation, food contact items and water contact items, films, coextrusion films, and exterior and interior automotive parts.

In certain embodiments, the substrate is a polymer selected from aliphatic and aromatic polyesters, such as polyethylene terephthalate (PET), polybutylene terephthalate, polyethylene isophthalate, polyhexamethylene terephthalate, polylactic acid, polyglycolic acid, and liquid crystalline polymers for high performance resins and fibers; polyester block copolymers; aliphatic and aromatic polyamides, such as nylon 6, nylon 66, nylon 610, nylon 11, nylon 12, nylon 1212, poly-p-phenylene terephthalamide, poly-m-phenylene isophthalamide; copolymerised polyamides; polyolefins such as polyethylene, polypropylene, and copolymers thereof; vinyl polymers such as polystyrene, polyacrylonitrile, polyvinylalcohol, polyvinyl acetate, polyvinylchloride, polyvinylidene chloride, ABS resins, and acrylic resins; copolymers of ethylene and vinyl acetate; fluorocarbon polymers such as polytetrafluoroethylene, polyvinylidene fluoride and polyvinyl fluoride; polyurethanes; segmented polyurethane elastomers, spandex or elastane elastomers; polyethers such as polyacetals; polyketones such as polyetherether ketone (PEEK) and polyether ketoneketone (PEKK); polyether and polyester block polymers; polysulfides; polysulfones: polysiloxanes such as polydimethyl siloxane; polycarbonates; thermosetting synthetic polymers such as phenol-formaldehyde copolymer, polyurethane, polyesterurethane, polyetherurethane, polyetherurethaneurea, and polyesterurethaneurea; natural polymers such as cellulosics, cotton and wool; and, regenerated or semi-synthetic polymers such as rayon, cuprammonium rayon, acetate rayon, triacetate rayon, reconstituted silk and polysaccharides. Copolymers, terpolymers, and blends of the polymer species listed are also contemplated.

In certain embodiments, at least a portion of the polymer is recycled polymer, for example, recycled post-consumer polymer waste. In certain embodiments, substantially all of the thermoplastic polymer is recycled. In certain embodiments, the recycled polymers are derived from polymer waste, for example, post-consumer polymer waste, post-industrial polymer waste, and/or post-agricultural waste polymer. In certain embodiments, the polymers are recycled post-consumer polymer waste.

In certain embodiments, the substrate is a polymeric film or laminate, for example, a polymeric film or film that may be processed into a container and/or tray and/or film for food grade packaging, for example, packaging for fresh produce such as, but not limited to, uncooked meats such as poultry, e.g., chicken, and cooked meats. In certain embodiments, the polymeric container or tray is formed of polypropylene or PET. Such containers and trays may be sealed with a flexible film of polyethylene or a polyester laminate. In certain embodiments, the substrate is a thermoforming film that may be processed into a container or tray for fresh produce. As discussed below, advantageously surface mineralization may be carried out during the thermoforming process.

In certain embodiments, the substrate is a cellulose-based substrate, for example, a paper product or textile.

In certain embodiments, the substrate is a nonwoven substrate. Surface-mineralized nonwoven substrates include products uses alone or as components of apparel, home furnishings, health care, engineering, industrial and consumer goods. Nonwoven products include solation gowns, surgical gowns, surgical drapes and covers, surgical masks, surgical scrub suits, caps, diaperstock, feminine hygiene, and other absorbent materials (e.g., sanitary wipes, baby wipes), carpet backing, primary and secondary, composites marine sail laminates, tablecover laminates, chopped strand mat, backing/stabilizer for machine embroidery, insulation, acoustic insulation for appliances, automotive components, and wall-paneling, pillows, cushions, mattress cores, and upholstery padding batting in quilts or comforters, consumer and medical face masks, mailing envelopes, tarps, tenting and transportation (lumber, steel) wrapping disposable clothing (foot coverings, coveralls), weather resistant house wrap, and cleanroom wipes. In certain embodiments, the nonwoven substrate is a wipe suitable for household use, for example, a sanitary wipe or baby wipe.

*Methods of manufacture*

In the following section, the method of manufacture may tend to be described in terms of an inorganic particulate material having an antibacterial capability. However, the invention should not be construed as being limited to such embodiments, i.e., the methods of manufacture are suitable for preparing a surface-mineralized substrate having any other of the functional capabilities described herein.

The surface-mineralized substrate may be made by a method comprising applying an inorganic particulate material having an antibacterial capability to the surface of a substrate, wherein the substrate has a surface region which is embeddable with the inorganic particulate material and/or a surface to which the inorganic particulate material is attachable, and wherein the inorganic particulate becomes embedded in the surface region of the substrate and/or attached to the surface of the substrate upon further processing.

The surface region is a region which extends from the surface into the body of the substrate by a distance no more than 10 % of the total thickness of the substrate, for example, no more than about 5 % of the total thickness of the substrate, or no more than about 2 % of the total thickness of the substrate, or no more than about 1 % of the total thickness of the substrate. In certain embodiments, the extent of the surface region is such that at least a portion of the inorganic particulate material having an antibacterial capability is partially, but not wholly, embedded in the surface of the substrate.

In certain embodiments, the surface region is a region which extends from the surface into the body of the substrate by a distance (i.e., measured perpendicular to the plane of the surface at the measurement point) of no more than about 20 µm, for example, no more than about 15 µm, or more than about 10 µm, or no more than about 5 µm, or no more than about 2 µm, or no more than about 1 µm.

In embodiments in which the substrate is a polymeric substrate, for example, a plastic substrate, the inorganic particulate material having antibacterial capability may be applied during manufacture of the substrate or following forming of the polymeric substrate, for example, during any suitable plastics shaping operation including compression molding, injection molding, thermoforming, calendaring and extrusion.

In such embodiments, the inorganic particulate material having an antibacterial capability may be applied to a surface of the substrate while the surface is in a softened state. For example, in a compression molding, injection molding or extrusion process, the inorganic particulate material having an antibacterial capability surface may be applied following molding/extrusion but before the molded/extruded piece has fully cooled. In other words, the temperature of the surface region of the substrate is such that the inorganic particulate material having an antibacterial capability becomes embedded in the surface region or at least attached to the surface. For example, the surface region of the substrate may be at a temperature at or above the melting point of the polymeric material of which the substrate is formed. In a laminate or layered substrate, the outermost, surface facing layer of polymeric material may have a melting temperature which is lower than the innermost layer or layers of the substrate, therefore providing an operating window in which the innermost layer or layers remain substantially solid whilst the surface region of the outermost layer is surface mineralized. Further processing of the substrate, e.g., by cooling and/or by the application of pressure to the surface (e.g., by using a roller) "locks-in" the inorganic particulate material having an antibacterial capability.

In other embodiments, the molded/extruded piece may be fully cooled and then heat applied to a surface portion to "re-soften" e.g., melt, the surface region and then inorganic particulate material having an antibacterial capability is applied to the surface. Additionally or alternatively, the inorganic particulate material having an antibacterial capability may be attached to a surface of the molded/extruded piece without having to soften or re-soften the surface, for example, by electrostatic attachment, between a surface of the substrate and the inorganic particulate material. In certain embodiments, the inorganic particulate material is attached to the surface electrostatically, e.g., by an electrostatic pinning method. Further, in certain embodiments, the inorganic particulate material may be positively or negatively charged, or modified such that it is positively or negatively charged, whilst the substrate is oppositely charged (or modified such that it is oppositely charged). This may enhance an even distribution of inorganic particulate material when applied and also enhance adherence of the inorganic particulate material to the surface of the surface prior to an embedding step.

In certain embodiments, the substrate is a thermoforming film and the inorganic particulate material having an antibacterial capability is applied to the surface of the polymeric film in a thermoforming process. Such a process for manufacturing packaging items is usually carried out using a roll of film, for example, an indexed roll of film, typically but not exclusively in a semi-continuous process, with a roll of film loaded at one end of the process and thermoformed containers, e.g., trays, produced at the end of the process. The trays are normally die cut from the film, and stacked at the end of the process. In certain embodiments, the process is a continuous process. In other embodiments, the process may be a batch process.

Typical steps in a thermoforming process are: 1) index film to heating position, 2) heat film (e.g., by IR), 3) index over mould, 4) pre-stretch, 5) vacuum or overpressure applied, 6) cooling, 7) index over die cutting, 8) die cutting, 9) ejection and stacking, and 10), index of matrix waste to rewind.

In certain embodiments, the inorganic particulate material having an antibacterial capability is applied to the thermoforming film prior to heating (which may be carried by IR treatment, but other heating techniques may be used), e.g., between steps 1) and 2) above. Alternatively, the inorganic material having an antibacterial capability may be applied following film heating, e.g., between steps 2) and 3). The application of the inorganic particulate material having an antibacterial capability can be done with or without masking, or by using automated (e.g., computerised) control to prevent or ameliorate inorganic particulate material falling in certain areas. An advantage of such a process is that once the thermoformed product is formed, the surface-mineralization is present at desired areas, for example, the base of a tray, and may not interfere with sealing or film clarity. In addition or alternatively, the inorganic particulate material having antibacterial capability can be electrostatically pinned to the plastic substrate. The thermoforming process may be completed with a prestretcher that applies pressure (and optionally additional temperature) into a desired area (e.g., the base of a tray) which facilitates embedding of the inorganic particulate material into the surface region of the substrate, prior to vacuum or over pressure stretching the thermoforming film into the corner regions of the mould.

In certain embodiments in which the film is printed then the surface-mineralization process can be carried out after printing and be done in register to the print (i.e., the inorganic particulate material having an antibacterial capability is applied to locate and register at defined areas of the substrate). An eye mark or electric eye may be used to register the application of the inorganic particulate material to the desired area(s). In this way, inorganic particulate material may be applied only to the back of areas where there is already print on the front side or encapsulated in a laminated film. In this way the inorganic particulate material may not adversely affect the clarity of the film as they are hidden by the print.

In certain embodiments, the inorganic particulate material having an antibacterial capability is itself heated prior to application, meaning that no heat, or at least less heat, may need to be applied directly to the thermoforming film, or to the injected/extruded substrate. The inorganic particulate material may need to be heated to a temperature greater than the melting temperature of the polymer to facilitate embedding on contact with the surface of the polymeric substrate. As with other methods of application, pressure may be applied following application (e.g., by a prestrecher as used in a thermoforming process, or using an optionally heated roller) to further enhance the embedding of the inorganic particulate material into the surface region of the substrate.

In certain embodiments, for example, embodiments in which the surface mineralization improves an adherence property of the substrate, the inorganic particulate material may be applied to the surface of a substrate via an intermediate transfer member. In certain embodiments, the intermediate transfer member is a surface of a machine part which the surface of the substrate contacts, and which transfers inorganic particulate material from the surface of the machine part to the surface of the substrate. Examples of machine parts include the internal surface of a mold in which the substrate is to be formed or processed. The surface of the intermediate transfer member, e.g., machine part, may be made attractive to the inorganic particulate material by static or by a coating that lightly adheres a layer of inorganic particulate material. The extent of adherence will be such that a least a portion, for example, at least 50 wt. % of the inorganic particulate material transfers from the intermediate transfer member to the substrate which is to be surface-mineralized. In certain embodiments, at least 75 wt. %, or at least 90 wt. %, or at least 95 wt. %, or at least 99 wt. %, or substantially all of the inorganic material is transferred to the surface of the substrate. The surface-mineralized substrate exhibiting an improved functional capability such as, for example, adherence, may then be brought in contact or together with another substrate, which may or may not be a like substrate, forming a composite, e.g., laminated or layered, structure. The presence of the surface mineralization on the substrate improves adherence between substrates.

In certain embodiments, an adherence property includes or is surface roughness and/or co-efficient of friction. Thus, in certain embodiments, surface mineralization of a surface of a substrate increases the surface roughness and/or co-efficient of friction of the surface. Thus, in certain embodiments, a surface of a substrate is roughened by application of the inorganic particulate material, in accordance with the methods described herein, such that friction between the surface and another surface is increased, for example, between a user (e.g., vehicle or subject) and the surface. Similarly, in certain embodiments, the co-efficient of friction of the surface is increased by application of the inorganic particulate material, in accordance with the methods described herein, such that friction between the surface and another surface is increased, for example, between a user (e.g., vehicle or subject) and the surface. In certain embodiments, the surface which is surface-mineralized to increase its roughness or co-efficient of friction is a region, or portion, or the entire surface of an article of manufacture. In certain embodiments, the article of manufacture is a road (e.g., motorway, highway, A-road, B-road, C-road, trunk road, minor road, and the like), pavement, path, walkway (including decking) or bridge deck (e.g., a deck for a vehicle- or foot-bridge), and the like. Such articles may be prefabricated. In certain embodiments, the article of manufacture is a section of, or an insert for, a road, pavement, path, walkway (including decking) or bridge deck, e.g., a flyover, or level crossing, or repair piece for a pothole, or cattle grid. In certain embodiments, the article of manufacture is a tire for a vehicle, or the sole of a shoe, or an anti-slip material which may be, for example, retrofitted to a surface. Roughening by surface-mineralization may be performed during manufacture of the article, e.g., during moulding or extruding the article.

For paper products, textiles and nonwovens and the like, the application of inorganic particulate material having an antibacterial may be applied during the forming process, in many ways analogously to polymeric substrate embodiments described above. For example, during manufacture of nonwovens, inorganic particulate material having an antibacterial capability may be applied prior to, during, or after, a final bonding step, for example, by spraying or blowing the inorganic particulate material having an inorganic particulate material at the emerging fibres or by dragging the emerging fibres through the inorganic particulate material. Paper products may be made by a similar process, for example, by applying (e.g., by spraying, blowing or dragging) inorganic particulate material to emerging fibres during manufacture of paper, followed by a finishing step, such as calendaring or supercalendaring step, to enhance the embedding of the inorganic particulate material into the surface region of the paper web.

According to any of the methods described above, the inorganic particulate material having an antibacterial capability may be applied by an suitable means of application. In certain embodiments, the inorganic particulate material having an antibacterial capability is applied by spraying and/or by gravimetric application, for example, using an offset sprayer/powder sprayer or a gravimetric feeder, or a combination thereof (e.g., a gravimetric feeder feeding a sprayer which applies the inorganic particulate material having an antibacterial agent to the surface of the substrate). Current manufacturing apparatus could be relatively easily adapted for carrying out the methods described herein by incorporating a sprayer and optionally a gravimetric feeder.

As described herein, certain embodiments enable a method for enhancing or improving the antibacterial activity of a substrate suitable for use in or as packaging for fresh produce, e.g., uncooked meat, by surface-mineralizing the substrate in accordance with one or more of the various methods described herein. Further, certain embodiments may enable a method for improving the shelf-life or consumer-appeal of fresh meat, for example, fresh poultry meat or red meat, by packaging fresh meat, for example, fresh poultry meat, in packaging comprising or formed from or of a surface-mineralized substrate according to one or more of the various aspects described herein.

By way of non-limiting illustration, examples of certain embodiments are given below:

**EXAMPLES**

Example 1

*Test method:*

Antibacterial activity ISO 22196:2007

*Experimental conditions:*

- Microorganisms: *Pseudomonas aeruginosa* ATCC® 15442™ (strain designation: PRD-10 [CIP 103467, NCIB 10421, PCI 812])
- Test inoculum: 0.4 ml with a bacterial concentration of $6 \times 10^5$ cells/ml, diluted in nutrient broth and physiological solution at 1/500 ratio.
- Contact time: 24 h at 37 $\pm$1°C.
- Neutralizing solution: 10 ml (30 g/l lecithin, 30 g/l Tween 80, 5 g/l sodium thiosulphate, 1 g/l L-histidine, 0.68 g/l $KH_2PO_4$, (pH a 7.2 $\pm$ 0.2)).
- Sterilization of the samples: no.
- Application of film: yes (50 mm x 50 mm).

The number of viable bacteria was calculated as follows:

N = (100 x C x D x V)/ A where:
N = number of viable bacteria recovered per $cm^2$ per test specimen.
C = average plate count for the duplicate plates.
D = dilution factor for the plates counted.
V = volume, in ml, of Neutralizing solution added to the specimen.
A = surface area, in $mm^2$, of the cover film ($2500 \ mm^2$).

The antibacterial activity, R, was calculated as follows:

$$R = (U_t - U_0) - (A_t - U_0) = U_t - A_t$$

where:

Uo = average of the common logarithm of the number of viable bacteria, in cells/$cm^2$, recovered from the untreated test specimens immediately after inoculation
Ut = average of the common logarithm of the number of viable bacteria, in cells/$cm^2$, recovered from the untreated test specimens after 24 h.
$A_t$ = average of the common logarithm of the number of viable bacteria, in cells/$cm^2$, recovered from the treated test specimens after 24 h.

*Test validation:*

- The number of viable cells immediately after inoculation: $(L_{max} - L_{min})/(L_{mean}) < 0.2$ where:

  - $L_{max}$ = logarithm of maximum number of viable cells counted at To
  - $L_{min}$ = logarithm of minimum number of viable cells counted at To
  - $L_{mean}$ = logarithm of the average number of viable cells in three test pieces counted at To

Obtained value: $(L_{max} - L_{min})/(L_{mean}) = 0.01$.

- The average of the number of viable cells immediately after inoculation on an untreated test piece shall be within the range of $6.2 \times 10^3$ cells/cm$^2$ to $2.5 \times 10^4$ cells/cm$^2$.

Obtained average value: $2.31 \times 10^4$ cells/cm$^2$.

- The number of viable cells on an untreated test piece after 24 h shall not be less than $6.2 \times 10^1$ cells/cm$^2$.

Obtained average value: $1.11 \times 10^4$ cells/cm$^2$.

Results:

| Sample | Pseudomonas aeruginosa ATCC® 15442™ | | |
|---|---|---|---|
| | cells/cm$^2$ (inoculated) T$_0$ | cells/cm$^2$ (recovered) 24 h | R |
| Reference | $21.9 \times 10^3$<br>$23.1 \times 10^3$<br>$24.4 \times 10^3$<br>Average $23.1 \times 10^3$ | $11.4 \times 10^3$<br>$11.4 \times 10^3$<br>$10.6 \times 10^3$<br>Average $11.1 \times 10^5$ | - |
| Sample 1 | $21.9 \times 10^3$<br>$23.1 \times 10^3$<br>$24.4 \times 10^3$<br>Average $23.1 \times 10^3$ | $78.4 \times 10^1$<br>$72.5 \times 10^1$<br>$75.0 \times 10^1$<br>Average $75.3 \times 10^1$ | 1.17 |
| Sample 2 | $21.9 \times 10^3$<br>$23.1 \times 10^3$<br>$24.4 \times 10^3$<br>Average $23.1 \times 10^3$ | $1.88 \times 10^0$<br>$1.25 \times 10^0$<br>$4.38 \times 10^0$<br>Average $2.5 \times 10^0$ | 3.65 |
| Sample 3 | $21.9 \times 10^3$<br>$23.1 \times 10^3$<br>$24.4 \times 10^3$<br>Average $23.1 \times 10^3$ | $25.6 \times 10^1$<br>$24.4 \times 10^1$<br>$19.4 \times 10^1$<br>Average $23.1 \times 10^1$ | 1.68 |
| Sample 4 | $21.9 \times 10^3$<br>$23.1 \times 10^3$<br>$24.4 \times 10^3$<br>Average $23.1 \times 10^3$ | $1.25 \times 10^0$<br>$1.25 \times 10^0$<br>$1.25 \times 10^0$<br>Average $1.25 \times 10^0$ | 3.95 |

- Reference = injection moulded polypropylene (PP) plaque
- Sample 1 = 1 wt. % silver doped diatomaceous earth compounded in before the PP plaque was injection moulded
- Sample 2 = 5 wt. % silver doped diatomaceous earth compounded in before the PP plaque was injected moulded
- Sample 3 = un-doped diatomaceous earth surface-mineralized onto the surface of the PP plaque (mineral content by ashing = 0.044 wt. %)
- Sample 4 = silver doped diatomaceous earth surface-mineralized onto the surface of the PP plaque (mineral content by ashing = 0.044 wt. %)

The ratio of R to wt. % of mineral in Sample 1 is 1.17.
The ratio of R to wt. % of mineral in Sample 2 is 0.73.
Sample 3 has an AAEF of 1.68/0.044 = 38.2.
Sample 4 has an AAEF of 3.95/0.044 = 89.9.
Based on these results it is seen that the antibacterial activity of the surface-mineralized plaques having a mineral content of just 0.044 wt. % is comparable to or better than the plaques having 5 wt. % mineral compounded into the plaque prior to injection moulding.

Example 2

The procedure in Example 1 was repeated for *Staphylococcus aureus* ATCC® 6538™ (strain designation: FDA 209).

*Test validation:*

- The number of viable cells immediately after inoculation: $(L_{max}-L_{min})/(L_{mean}) < 0.2$ where:

  - $L_{max}$ = logarithm of maximum number of viable cells counted at To
  - $L_{min}$ = logarithm of minimum number of viable cells counted at To
  - $L_{mean}$ = logarithm of the average number of viable cells in three test pieces counted at To

Obtained value: $(L_{max}-L_{min})/(L_{mean}) = 0.02$.

- The average of the number of viable cells immediately after inoculation on an untreated test piece shall be within the range of $6.2 \times 10^3$ cells/cm$^2$ to $2.5 \times 10^4$ cells/cm$^2$.

Obtained average value: $2.31 \times 10^4$ cells/cm$^2$.

- The number of viable cells on an untreated test piece after 24 h shall not be less than $6.2 \times 10^1$ cells/cm$^2$.

Obtained average value: $2.38 \times 10^6$ cells/cm$^2$.

*Results:*

| Sample | *Staphylococcus aureus* ATCC® 6538™ | | |
|---|---|---|---|
| | cells/cm$^2$ (inoculated) $T_0$ | cells/cm$^2$ (recovered) 24 h | R |
| Reference | $20.6 \times 10^3$<br>$23.8 \times 10^3$<br>$25.0 \times 10^3$<br>Average $23.1 \times 10^3$ | $21.3 \times 10^5$<br>$26.3 \times 10^5$<br>$23.8 \times 10^5$<br>Average $23.8 \times 10^5$ | - |
| Sample 1 | $21.3 \times 10^3$<br>$22.5 \times 10^3$<br>$21.9 \times 10^3$<br>Average $21.9 \times 10^3$ | $10.8 \times 10^3$<br>$12.5 \times 10^3$<br>$10.0 \times 10^3$<br>Average $11.1 \times 10^3$ | 2.33 |
| Sample 2 | $20.6 \times 10^3$<br>$23.1 \times 10^3$<br>$20.0 \times 10^3$<br>Average $21.3 \times 10^3$ | $6.25 \times 10^0$<br>$6.25 \times 10^0$<br>$6.25 \times 10^0$<br>Average $6.25 \times 10^0$ | 5.58 |
| Sample 3 | $20.6 \times 10^3$<br>$24.4 \times 10^3$<br>$21.3 \times 10^3$<br>Average $22.1 \times 10^3$ | $25.0 \times 10^2$<br>$22.5 \times 10^2$<br>$23.8 \times 10^2$<br>Average $23.8 \times 10^2$ | 3.00 |
| Sample 4 | $21.9 \times 10^3$<br>$24.4 \times 10^3$<br>$19.4 \times 10^3$<br>Average $21.9 \times 10^3$ | $6.25 \times 10^0$<br>$6.25 \times 10^0$<br>$6.25 \times 10^0$<br>Average $6.25 \times 10^0$ | 5.58 |

The ratio of R to wt. % of mineral in Sample 1 is 2.33.

The ratio of R to wt. % of mineral in Sample 2 is 1.16.

Sample 3 has an AAEF of 3.00/0.044 = 68.2.

Sample 4 has an AAEF of 5.58/0.044 = 126.8.

Based on these results it is seen that the antibacterial activity of the surface-mineralized plaques having a mineral content of just 0.044 wt. % is comparable to or better than the plaques having 5 wt. % mineral compounded into the plaque prior to injection moulding.

**Claims**

1. A surface-mineralized substrate having enhanced antibacterial activity, the substrate comprising an inorganic particulate material embedded in a surface region thereof and/or attached to a surface thereof, wherein the inorganic particulate material has an antibacterial capability, wherein the surface-mineralized substrate has an antibacterial activity enhancement factor (AAEF) of at least 1.5,
   wherein AAEF = R/W, where

   R is the antibacterial activity of the surface-mineralized substrate according to ISO 22196:2007 for *Pseudomonas aeruginosa,*
   W is the % by weight of inorganic particulate material embedded in the surface region and/or attached to the surface of the surface-mineralized substrate, based on the total weight of the surface mineralized substrate;
   wherein W is from 0.01 to 0.25,
   wherein the surface region is a region extending from the surface of the substrate into the body of the substrate, and
   wherein the surface region constitutes no more than 10 % of the total thickness of the substrate,
   wherein the inorganic particulate material is associated with an antibacterial agent, the antibacterial agent being silver.

2. A surface-mineralized substrate having enhanced antibacterial activity, the substrate comprising an inorganic particulate material embedded in a surface region thereof and/or attached to a surface thereof, wherein the inorganic particulate material has an antibacterial capability, and wherein the surface-mineralized substrate has an antibacterial activity, R, of at least about 3.0 according to ISO 22196:2007 for *Pseudomonas aeruginosa* when the substrate comprises from 0.02 to 0.10 % by weight of inorganic particulate material in the surface region thereof and/or attached to the surface thereof, based on the total weight of the surface-mineralized substrate,

   wherein the surface-mineralized substrate comprises from 0.02 to 0.10 % by weight of inorganic particulate material in the surface region thereof and/or attached to the surface thereof, based on the total weight of the surface-mineralized substrate,
   wherein the surface region is a region extending from the surface of the substrate into the body of the substrate, and
   wherein the surface region constitutes no more than 10 % of the total thickness of the substrate,
   wherein the inorganic particulate material is associated with an antibacterial agent, the antibacterial agent being silver.

3. A surface-mineralized substrate having an inorganic particulate material embedded in a surface region thereof and/or attached to a surface thereof, wherein the surface-mineralized substrate has at least one functional capability, wherein the substrate comprises 0.01 to 0.25 % by weight of the inorganic particulate material in the surface region thereof and/or attached to the surface thereof, wherein the at least one functional capability is selected from:

   biocidal (including herbicidal, fungicidal and/or pesticdial) activity; and/or
   biocide (including herbicide, fungicide and pesticide) release,

   wherein the at least one functional capability is a property of the inorganic particulate and/or is a property of an active agent associated with the inorganic particulate material in the surface region thereof and/or attached to the surface thereof,,

   wherein the surface region is a region extending from the surface of the substrate into the body of the substrate, and

wherein the surface region constitutes no more than 10 % of the total thickness of the substrate,
wherein the inorganic particulate material is associated with an antibacterial agent, the antibacterial agent being silver.

4. A surface-mineralized substrate according to any preceding claim, wherein the inorganic particulate material having an antibacterial capability is electrostatically attached to the surface of the substrate.

5. A surface-mineralized substrate according to claim 1 or any claim dependent thereon having an AAEF of at least about 5, for example, at least about 50, or at least about 100.

6. A surface-mineralised substrate according to any preceding claim, wherein R is at least 5.0., optionally wherein W or the % by weight of inorganic particulate material having an antibacterial capability is from 0.02 to 0.08.

7. A surface-mineralized substrate according to any preceding claim, wherein the antibacterial agent is present in an amount of no more than about 10 % by weight, based on the weight of the of the inorganic particulate material.

8. A surface-mineralized substrate according to any preceding claim, wherein the inorganic particulate material having an antibacterial capability is selected from the group consisting of diatomaceous earth, kaolin, mica, vermiculite, perlite, talc, zeolite, and aluminosilicates.

9. A surface-mineralised substrate according to claim 8, wherein the inorganic particulate material is diatomaceous earth.

10. A surface-mineralized substrate according to any preceding claim, wherein the substrate is a polymeric substrate, for example, a plastic, or a polymeric film or laminate.

11. A surface-mineralized substrate according to any one of claims 1-9, wherein the substrate is cellulose-based substrate, for example, paper, or a non-woven substrate.

12. An article of manufacture, for example, packaging for fresh produce, comprising or formed from or of a surface-mineralized substrate according to any preceding claim, optionally wherein the packaging comprises a container for housing the fresh produce which is sealable with a polymeric film, wherein at least a portion of the container and/or the polymeric film is a surface-mineralized substrate.

13. A packaged product comprising the packaging according to claim 12, further comprising a carbon dioxide-producing component within the packaging, optionally wherein the carbon dioxide-producing component is associated with the surface-mineralized substrate, for example, embedded in the surface region thereof and/or attached to a surface thereof.

14. A method suitable for making a surface-mineralized substrate according to any one of claims 1, 2 or 3, the method comprising applying an inorganic particulate material having an antibacterial capability to the surface of a substrate, wherein the substrate has a surface region which is embeddable with the inorganic particulate material and/or a surface to which the inorganic particulate material is attachable, and wherein the inorganic particulate becomes embedded in the surface region of the substrate and/or attached to the surface of the substrate upon further processing.

15. A method according to claim 14, wherein the substrate is a thermoforming film and the inorganic particulate material having an antibacterial capability is applied to the surface of the film in a thermoforming process, optionally prior to heating the film for moulding.

16. A method for improving the antibacterial activity of a substrate suitable for use in or as packaging for fresh produce, said method comprising surface-mineralizing the substrate in accordance with the method according to any one of claims 14-15.

17. A method for improving the shelf-life of fresh poultry meat, said method comprising packaging fresh poultry meat in packaging comprising or formed from or of a surface-mineralized substrate according to any one of claims 1-11.

**Patentansprüche**

1. Oberflächenmineralisiertes Substrat mit erhöhter antibakterieller Aktivität, wobei das Substrat in einem seiner Oberflächenbereiche eingebettete und/oder anhaftende anorganische Partikel aufweist, wobei die anorganischen Partikel eine antibakterielle Wirkung aufweisen, wobei das oberflächenmineralisierte Substrat einen Antibakteriellen Aktivitätsverstärkungsfaktor (AAVF) von mindestens 1,5 aufweist, wobei AAVF = R/W ist, wobei

R die antibakterielle Aktivität des oberflächenmineralisierten Substrats gemäß ISO 22196:2007 für *Pseudomonas aeruginosa* ist,
W der Gewichtsprozentsatz von im Oberflächenbereich des oberflächenmineralisierten Substrats eingebetteten und/oder angehafteten anorganischen Partikeln bezogen auf das Gesamtgewicht des oberflächenmineralisierten Substrats ist;
wobei W von 0,01 bis 0,25 ist,
wobei der Oberflächenbereich ein Bereich ist, der sich von der Oberfläche des Substrats bis in den Körper des Substrats erstreckt, und
wobei der Oberflächenbereich nicht mehr als 10% der Gesamtdicke des Substrats ausmacht,
wobei die anorganischen Partikel mit einem antibakteriellen Mittel assoziiert sind, wobei das antibakterielle Mittel Silber ist.

2. Oberflächenmineralisiertes Substrat mit erhöhter antibakterieller Aktivität, wobei das Substrat in einem seiner Oberflächenbereiche eingebettete und/oder anhaftende anorganische Partikel aufweist, wobei die anorganischen Partikel eine antibakterielle Wirkung aufweisen und wobei das oberflächenmineralisierte Substrat einen Antibakteriellen Aktivitätsverstärkungsfaktor (R) von mindestens 3,0 gemäß ISO 22196:2007 für *Pseudomonas aeruginosa* aufweist, wenn das Substrat - bezogen auf das Gesamtgewicht des oberflächenmineralisierten Substrats - in einem Oberflächenbereich 0,02 bis 0,10 Gewichtsprozent anorganische Partikel enthält und/oder anhaften hat,
wobei das oberflächenmineralisierte Substrat - bezogen auf das Gesamtgewicht des oberflächenmineralisierten Substrats - 0,02 bis 0,10 Gewichtsprozent anorganische Partikel in einem Oberflächenbereich enthält und/oder anhaften hat,
wobei der Oberflächenbereich ein Bereich ist, der sich von der Oberfläche des Substrats bis in den Körper des Substrats erstreckt, und
wobei der Oberflächenbereich nicht mehr als 10% der Gesamtdicke des Substrats ausmacht,
wobei die anorganischen Partikel mit einem antibakteriellen Mittel assoziiert sind, wobei das antibakterielle Mittel Silber ist.

3. Oberflächenmineralisiertes Substrat mit in einem Oberflächenbereich eingebetteten und/oder anhaftenden anorganischen Partikeln, wobei das oberflächenmineralisierte Substrat mindestens eine funktionelle Wirkung aufweist, wobei das Substrat im Oberflächenbereich 0,01 bis 0,25 Gewichtsprozent anorganische Partikel enthält und/oder anhaften hat und wobei die mindestens eine funktionelle Wirkung ausgewählt wird aus:
bioziden (einschließlich herbiziden, fungiziden und/oder pestiziden) Aktivitäten; und/oder bioziden (einschließlich herbiziden, fungiziden und/oder pestiziden) Freisetzungen,
wobei die mindestens eine funktionelle Wirkung eine Eigenschaft der anorganischen Partikel und/oder eine Eigenschaft eines Wirkstoffs ist, der mit den anorganischen Partikeln im Oberflächenbereich assoziiert ist und/oder an dessen Oberfläche anhaftet.
wobei der Oberflächenbereich ein Bereich ist, der sich von der Oberfläche des Substrats bis in den Körper des Substrats erstreckt und
wobei der Oberflächenbereich nicht mehr als 10% der Gesamtdicke des Substrats ausmacht,
wobei die anorganischen Partikel mit einem antibakteriellen Mittel assoziiert sind, wobei das antibakterielle Mittel Silber ist.

4. Oberflächenmineralisiertes Substrat gemäß einem der vorhergehenden Ansprüche, wobei die anorganischen Partikel mit antibakterieller Wirkung elektrostatisch an die Oberfläche des Substrats gebunden sind.

5. Oberflächenmineralisiertes Substrat gemäß Anspruch 1 oder einem davon abhängigen Anspruch mit einem AAVF von mindestens etwa 5, zum Beispiel, mindestens etwa 50 oder mindestens etwa 100.

6. Oberflächenmineralisiertes Substrat gemäß einem der vorhergehenden Ansprüche, wobei R mindestens 5,0 ist und wobei W oder der Gewichtsprozentsatz der anorganischen Partikel mit antibakterieller Wirkung gegebenenfalls 0,02 bis 0,08 beträgt.

7. Oberflächenmineralisiertes Substrat gemäß einem der vorhergehenden Ansprüche, wobei das antibakterielle Mittel - bezogen auf das Gewicht der anorganischen Partikel - in einer Menge von nicht mehr als etwa 10% vorhanden ist.

8. Oberflächenmineralisiertes Substrat gemäß einem der vorhergehenden Ansprüche, wobei die anorganischen Partikel mit antibakterieller Wirkung ausgewählt sind aus der Gruppe bestehend aus Kieselgur, Kaolin, Glimmer, Vermiculit, Perlit, Talk, Zeolith und Aluminosilicaten.

9. Oberflächenmineralisiertes Substrat gemäß Anspruch 8, wobei die anorganischen Partikel Kieselgur sind.

10. Oberflächenmineralisiertes Substrat gemäß einem der vorhergehenden Ansprüche, wobei das Substrat ein polymeres Substrat ist, beispielsweise ein Kunststoff, ein Polymerfilm oder eine Kunststoff-Folie.

11. Oberflächenmineralisiertes Substrat gemäß einem der Ansprüche 1 bis 9, wobei das Substrat ein auf Cellulose basiertes Substrat wie Papier oder ein Vliesträgermaterial ist.

12. Fabrikerzeugnis, beispielsweise eine Verpackung für Frischware, das ein oberflächenmineralisiertes Substrat aufweist oder aus einem oberflächenmineralisierten Substrat gemäß einem der vorhergehenden Ansprüche gebildet ist, wobei die Verpackung gegebenenfalls einen Behälter zur Aufnahme der Frischware umfasst, der mit einem Polymerfilm versiegelt werden kann, wobei mindestens ein Teil des Behälters und/oder des Polymerfilms ein oberflächenmineralisiertes Substrat ist.

13. Abgepacktes Produkt, umfassend die Verpackung gemäß Anspruch 12, ferner umfassend eine in der Verpackung befindliche Kohlendioxid-produzierende Komponente, wobei die Kohlendioxid-produzierende Komponente gegebenenfalls mit dem oberflächenmineralisierten Substrat verbunden ist, beispielsweise im Oberflächenbereich eingebettet ist und/oder am Oberflächenbereich anhaftet.

14. Verfahren, das sich zur Herstellung eines oberflächenmineralisierten Substrats gemäß einem der Ansprüche 1, 2 oder 3 eignet, wobei das Verfahren umfasst, anorganische Partikel mit antibakterieller Wirkung auf der Oberfläche eines Substrats anbringen, wobei das Substrat einen Oberflächenbereich aufweist, in den die anorganischen Partikel eingebettet werden können, und/oder einen Oberflächenbereich aufweist, an den die anorganischen Partikel aufgebracht werden können, und wobei die anorganischen Partikel im Laufe der weiteren Verarbeitung in den Oberflächenbereich eingebettet und/oder an der Oberfläche des Substrats angehaftet werden.

15. Verfahren gemäß Anspruch 14, wobei das Substrat eine warmgeformte Folie ist und die anorganischen Partikel mit antibakterieller Wirkung in einem Thermoformverfahren, gegebenenfalls vor dem Warmformen der Folie, auf die Oberfläche der Folie aufgebracht werden.

16. Verfahren, die antibakterielle Wirkung eines Substrats zu verbessern, das als Verpackung für Frischwaren eingesetzt werden soll, wobei das Verfahren umfasst, die Oberfläche des Substrat gemäß einem Verfahren gemäß einem der Ansprüche 14 bis 15 zu mineralisieren.

17. Verfahren zur Verbesserung der Haltbarkeit von frischem Geflügelfleisch, wobei das Verfahren umfasst, das frische Geflügelfleisch in eine Verpackung zu verpacken, die ein oberflächenmineralisiertes Substrat gemäß einem der Ansprüche 1 bis 11 aufweist oder aus diesem oberflächenmineralisierten Substrat geformt wurde.

**Revendications**

1. Un substrat minéralisé en surface ayant une activité antibactérienne améliorée, le substrat comprenant un matériau particulaire inorganique intégré dans une région de surface de celui-ci et / ou fixé à une surface de celui-ci, dans lequel le matériau particulaire inorganique a une capacité antibactérienne, dans lequel le substrat minéralisé en surface a un facteur d'amélioration d'activité antibactérien (AAEF) d'au moins 1,5, dans lequel AAEF = R/W, où

R est l'activité antibactérienne du substrat minéralisé en surface selon la norme ISO 22196:2007 pour *Pseudomonas aeruginosa,*

W est le % en poids de matériau particulaire inorganique intégré dans la région de surface et / ou fixé à la surface du substrat minéralisé en surface, sur la base du poids total du substrat minéralisé en surface ;

dans lequel W est de 0,01 à 0,25,

dans lequel la région de surface est une région s'étendant de la surface du substrat dans le corps du substrat, et

dans lequel la région de surface ne constitue pas plus de 10 % de l'épaisseur totale du substrat,

dans lequel le matériau particulaire inorganique est associé à un agent antibactérien, l'agent antibactérien étant l'argent.

**2.** Un substrat minéralisé en surface ayant une activité antibactérienne améliorée, le substrat comprenant un matériau particulaire inorganique intégré dans une région de surface de celui-ci et / ou fixé à une surface de celui-ci, dans lequel le matériau particulaire inorganique a une capacité antibactérienne, et dans lequel le substrat minéralisé en surface a une activité antibactérienne, R, d'au moins environ 3,0 selon la norme ISO 22196:2007 pour *Pseudomonas aeruginosa* lorsque le substrat comprend de 0,02 à 0,10 % en poids de matériau particulaire inorganique dans la région de surface de celui-ci et / ou fixé à la surface de celui-ci, sur la base du poids total du substrat minéralisé en surface,

dans lequel le substrat minéralisé en surface comprend de 0,02 à 0,10 % en poids de matériau particulaire inorganique dans la région de surface de celui-ci et / ou fixé à la surface de celui-ci, sur la base du poids total du substrat minéralisé en surface, dans lequel la région de surface est une région s'étendant de la surface du substrat dans le corps du substrat, et

dans lequel la région de surface ne constitue pas plus de 10 % de l'épaisseur totale du substrat,

dans lequel le matériau particulaire inorganique est associé à un agent antibactérien, l'agent antibactérien étant l'argent.

**3.** Un substrat minéralisé en surface ayant un matériau particulaire inorganique intégré dans une région de surface de celui-ci et / ou fixé à une surface de celui-ci, dans lequel le substrat minéralisé en surface a au moins une capacité fonctionnelle, dans lequel le substrat comprend 0,01 à 0,25 % en poids du matériau particulaire inorganique dans la région de surface de celui-ci et / ou fixé à la surface de celui-ci, dans lequel l'au moins une capacité fonctionnelle est choisie parmi :

l'activité biocide (y compris herbicide, fongicide et / ou pesticide) ; et / ou

la libération du biocide (y compris herbicide, fongicide et pesticide),

dans lequel l'au moins une capacité fonctionnelle est une propriété de la particule inorganique et / ou est une propriété d'un agent actif associé au matériau particulaire inorganique dans la région de surface de celui-ci et / ou fixé à la surface de celui-ci,

dans lequel la région de surface est une région s'étendant de la surface du substrat dans le corps du substrat, et

dans lequel la région de surface ne constitue pas plus de 10 % de l'épaisseur totale du substrat,

dans lequel le matériau particulaire inorganique est associé à un agent antibactérien, l'agent antibactérien étant l'argent.

**4.** Un substrat minéralisé en surface selon l'une quelconque des revendications précédentes, dans lequel le matériau particulaire inorganique ayant une capacité antibactérienne est fixé de manière électrostatique à la surface du substrat.

**5.** Un substrat minéralisé en surface selon la revendication 1 ou l'une quelconque des revendications qui dépend de celle-ci ayant un AAEF d'au moins environ 5, par exemple, d'au moins environ 50, ou d'au moins environ 100.

**6.** Un substrat minéralisé en surface selon l'une quelconque des revendications précédentes, dans lequel R est au moins 5,0, éventuellement dans lequel W ou le % en poids d'un matériau particulaire inorganique ayant une capacité antibactérienne est de 0,02 à 0,08.

**7.** Un substrat minéralisé en surface selon l'une quelconque des revendications précédentes, dans lequel l'agent antibactérien est présent en une quantité n'excédant pas environ 10 % en poids, sur la base du poids du matériau particulaire inorganique.

**8.** Un substrat minéralisé en surface selon l'une quelconque des revendications précédentes, dans lequel le matériau particulaire inorganique ayant une capacité antibactérienne est choisi parmi le groupe constitué de la terre de diatomées, du kaolin, du mica, de la vermiculite, de la perlite, du talc, de la zéolite et des aluminosilicates.

9. Un substrat minéralisé en surface selon la revendication 8, dans lequel le matériau particulaire inorganique est de la terre de diatomées.

10. Un substrat minéralisé en surface selon l'une quelconque des revendications précédentes, dans lequel le substrat est un substrat polymère, par exemple, un plastique, ou un film ou stratifié polymère.

11. Un substrat minéralisé en surface selon l'une quelconque des revendications 1 à 9, dans lequel le substrat est un substrat à base de cellulose, par exemple, du papier, ou un substrat non tissé.

12. Un article de fabrication, par exemple, un emballage pour produits frais, comprenant ou formé à partir ou d'un substrat minéralisé en surface selon l'une quelconque des revendications précédentes, éventuellement dans lequel l'emballage comprend un récipient pour loger les produits frais qui est scellable avec un film polymère, dans lequel au moins une partie du récipient et / ou du film polymère est un substrat minéralisé en surface.

13. Un produit emballé comprenant l'emballage selon la revendication 12, comprenant en outre un composant producteur de dioxyde de carbone à l'intérieur de l'emballage, éventuellement dans lequel le composant producteur de dioxyde de carbone est associé au substrat minéralisé en surface, par exemple, intégré dans la région de surface de celui-ci et / ou fixé à une surface de celui-ci.

14. Un procédé approprié pour fabriquer un substrat minéralisé en surface selon l'une quelconque des revendications 1, 2 ou 3, le procédé comprenant l'application d'un matériau particulaire inorganique ayant une capacité antibactérienne à la surface d'un substrat, dans lequel le substrat a une région de surface qui peut être intégrée au matériau particulaire inorganique et / ou une surface à laquelle le matériau particulaire inorganique peut être fixé, et dans lequel la particule inorganique s'intègre dans la région de surface du substrat et / ou se fixe à la surface du substrat après traitement ultérieur.

15. Un procédé selon la revendication 14, dans lequel le substrat est un film thermoformé et le matériau particulaire inorganique ayant une capacité antibactérienne est appliqué sur la surface du film dans un processus de thermoformage, éventuellement avant de chauffer le film pour moulage.

16. Un procédé pour améliorer l'activité antibactérienne d'un substrat approprié pour utilisation dans ou comme emballage pour des produits frais, ledit procédé comprenant la minéralisation de surface du substrat conformément au procédé selon l'une quelconque des revendications 14 à 15.

17. Un procédé pour améliorer la durée de conservation de la viande de volaille fraîche, ledit procédé comprenant l'emballage de viande de volaille fraîche dans un emballage comprenant, formé à partir ou formé d'un substrat minéralisé en surface selon l'une quelconque des revendications 1 à 11.

FIGURE 1

EP 3 240 404 B1

**EP 3 240 404 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100260866 A

- WO 2008150867 A